# EUROPEAN PATENT APPLICATION

(11) **EP 4 756 012 A1**
(43) Date of publication of application: **10.06.2026**
(21) Application number: 24855758.9
(22) Date of filing: 19.08.2024
(51) Int. Cl.: C12N 5/00, C12M 1/00

(54) **METHOD, DEVICE, SYSTEM AND MEDIUM FOR TREATING CELL PREPARATION**

(30) Priority: 22.08.2023 CN 202311064991
(71) Applicant: Shenzhen Cellbri Bio-Innovation Technology Co., Ltd., Shenzhen, Guangdong 518107 (CN)
(72) Inventor: GUO, Xiaoliang, Shenzhen, Guangdong 518107 (CN); SHANG, Yuanfang, Shenzhen, Guangdong 518107 (CN)
(74) Representative: Herrero & Asociados, S.L.
(86) International application number: PCT/CN2024/112957
(87) International publication number: WO 2025/040035

(57) **Abstract**

A cell preparation processing method, system, and storage medium are disclosed. The method includes: firstly, performing cell expansion processing on a to-be-cultured cell suspension in a cell culture container to obtain a cultured cell suspension; secondly, performing washing and concentration processing on the cultured cell suspension in the cell culture container to obtain a concentrated cell suspension; and thirdly, dispensing the concentrated cell suspension from the cell culture container into a plurality of preparation bags. By integrating the cell culture, the cell washing and concentration processing, and the preparation dispensing all within the same cell culture container, the present disclosure avoids dispersed arranged of the devices and reduces space occupation. By performing these steps sequentially within the same cell culture container, the entire process from the cell culture to the cell preparation can be carried out in a sealed sterile environment without the need for cell suspension transfer, thereby improving the quality of the cell culture. Furthermore, this sequential and integrated process reduces manual involvement and enables intelligent execution from the cell culture to the preparation dispensing, significantly improving the efficiency of the cell culture.

## Description

The present disclosure claims priority to the Chinese patent application with application number 202311064991.5, titled "Cell preparation processing method, device, system, and medium", filed on August 22, 2023, the entire contents of which are incorporated herein by reference into the present disclosure.

### TECHNICAL FIELD

The present disclosure relates to the field of biological processing technology, and more particularly, to a cell preparation processing method, device, system, and storage medium.

### BACKGROUND

Cell therapy is a key development area at the forefront of international medicine. The process of the cell therapy involves multiple critical steps such as cell culture, cell processing, and cell preparation. Currently, in the process of the cell therapy, these steps are often carried out in separate areas, that is, these steps are achieved through manual operations within each respective zones. The inventor has realized that a single step may require using different devices located in different zones. However, the dispersed arrangement of the devices occupies significant space and demands a high degree of manual involvement. In practice, operational errors are prone to occur in any of these steps due to variations in personnel experience, techniques, and habits, which can subsequently impact the quality and efficiency of the cell culture. Therefore, how to achieve an integrated process for the cell culture, the cell processing, and the cell preparation presents an urgent challenge for those skilled in the art.

### SUMMARY OF THE DISCLOSURE

The embodiments of the present disclosure provide a cell preparation processing method, device, system, and storage medium, aiming to solve the problems of high manual involvement and low automation of device in the existing technology, which results in a low cell culture quality and efficiency.

A cell preparation processing method, including:
performing cell expansion processing on a to-be-cultured cell suspension in a cell culture container to obtain a cultured cell suspension;
performing washing and concentration processing on the cultured cell suspension in the cell culture container to obtain a concentrated cell suspension; and
dispensing the concentrated cell suspension from the cell culture container into a plurality of preparation bags.

A cell preparation processing device, including:
a cell expansion module, configured to perform cell expansion processing on a to-be-cultured cell suspension in a cell culture container to obtain a cultured cell suspension;
a washing and concentration module, configured to perform washing and concentration processing on the cultured cell suspension in the cell culture container to obtain a concentrated cell suspension; and
a first preparation dispensing module, configured to dispense the concentrated cell suspension from the cell culture container into a plurality of preparation bags.

A cell preparation processing method system, including a cell culture container and a controller connected to the cell culture container, wherein the controller is configured to execute the following cell preparation processing method:
performing cell expansion processing on a to-be-cultured cell suspension in a cell culture container to obtain a cultured cell suspension;
performing washing and concentration processing on the cultured cell suspension in the cell culture container to obtain a concentrated cell suspension; and
dispensing the concentrated cell suspension from the cell culture container into a plurality of preparation bags.

One or more computer-readable storage media storing computer-readable instructions, wherein when the computer-readable instructions are executed by one or more processors, the one or more processors are caused to perform the following steps:
performing cell expansion processing on a to-be-cultured cell suspension in a cell culture container to obtain a cultured cell suspension;
performing washing and concentration processing on the cultured cell suspension in the cell culture container to obtain a concentrated cell suspension; and
dispensing the concentrated cell suspension from the cell culture container into a plurality of preparation bags.

The above cell preparation processing method, device, system, and storage medium integrate the cell culture, the cell washing and concentration processing, and the preparation dispensing within the same cell culture container, which avoids dispersed arrangement of the devices and thus reduces space occupation. By performing these steps sequentially within the same cell culture container, the entire process from the cell culture to the cell preparation can be carried out in a sealed sterile environment without the need for cell suspension transfer, thereby improving the quality of the cell culture. Furthermore, this sequential and integrated process reduces manual involvement and enables intelligent execution from the cell culture to the preparation dispensing, significantly improving the efficiency of the cell culture.

The details of one or more embodiments of the present disclosure are presented in the accompanying drawings and description below, and other features and advantages of the present disclosure will become apparent from the specification, drawings, and claims.

### BRIEF DESCRIPTION OF THE DRAWINGS

To illustrate the technical solutions in this invention or the prior art more clearly, a brief introduction to the drawings required for describing the embodiments or prior art will be provided below. It is evident that the drawings described below are some embodiments of this invention. For those skilled in the art, other drawings can be obtained based on these drawings without requiring creative effort.
FIG. 1 is a flowchart of a cell preparation processing method in accordance with an embodiment of the present disclosure;
FIG. 2 is a schematic diagram of a cell preparation processing device in accordance with an embodiment of the present disclosure; and
FIG. 3 is a schematic diagram of a cell preparation processing system in accordance with an embodiment of the present disclosure.

The accompanying figures in the manual are labeled as follows:
1, sample bag; 2, washing solution bag; 3, replacement solution bag; 4, preparation bag; 5, intermediate product bag; 6, culture medium; 7, on-off valve; 8, bubble sensor; 9, pressure sensor; 10, peristaltic pump; 11, quantitative tube; 12, cell culture container.

### PREFERRED EMBODIMENTS

To make the objectives, technical solutions, and advantages of the present disclosure clearer, the technical solutions of the present disclosure will be clearly and comprehensively described below in conjunction with the accompanying drawings. It is apparent that the described embodiments are a part of the embodiments of the present disclosure, not all embodiments. Based on the embodiments of the present disclosure, all other embodiments obtained by those of ordinary skill in the art without creative effort fall within the scope of protection of the present disclosure.

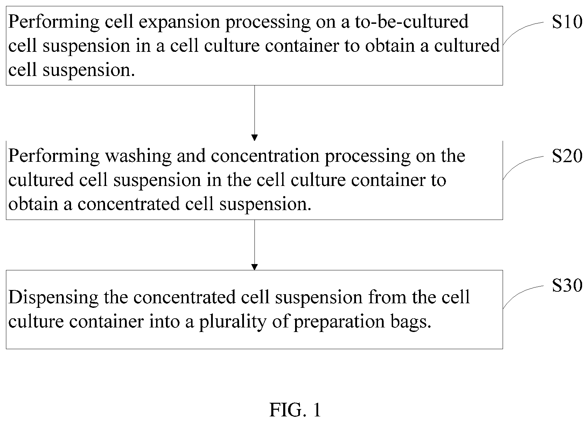

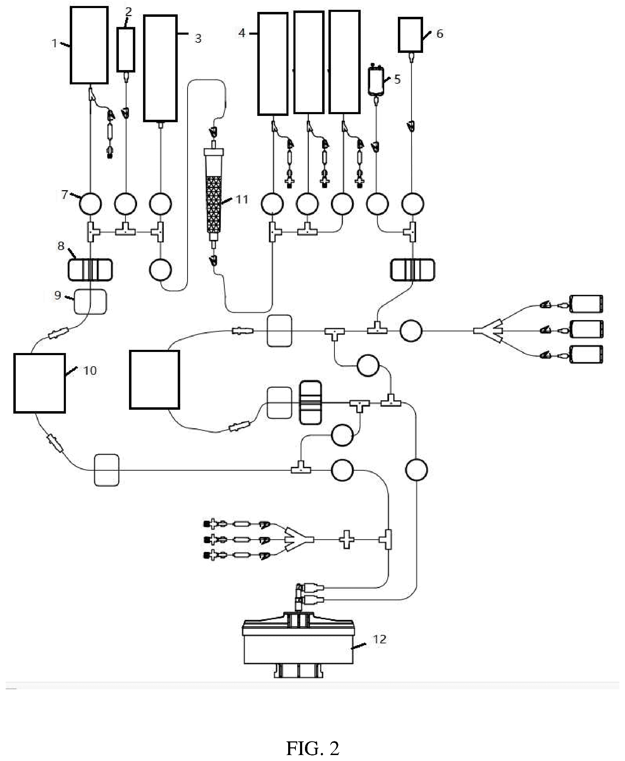

In an embodiment, as shown in FIG. 1 and FIG. 2, a cell preparation processing method is provided, including steps as follows.

S10, performing cell expansion processing on a to-be-cultured cell suspension in a cell culture container to obtain a cultured cell suspension.

It is understood that a bag containing the to-be-cultured cell suspension (which can be a sample bag 1 in FIG. 2) can be connected to an inlet/outlet of the cell culture container. The to-be-cultured cell suspension is then transferred into the cell culture container along a connected tubing path through a delivery device (such as a peristaltic pump). The to-be-cultured cell suspension may include T cells and T cell subsets. The T cells, also known as T lymphocytes, are a type of pluripotent stem cell derived from bone marrow. The T cell subsets are classifications of the T cells based on different classification methods; that is, a T subset is one classifications under the T cells. The cell expansion processing is used to induce the proliferation of the to-be-cultured cell suspension, thereby achieving a large-scale expansion of the T cells and the T cell subsets to meet a cell number required for cell therapy. The cultured cell suspension contains the T cells, the T cell subsets, and non-cellular impurities (i.e., impurities generated during the cell expansion processing). The T cells in the to-be-cultured cell suspension can be obtained by differentiating T cell progenitors. During the cell expansion processing, a culture nutrient solution can be introduced into the cell culture container. The culture nutrient solution can be a culture medium, which is an artificial nutrient used for cultivating and propagating microorganisms, cells, and tissues. The culture nutrient solution can be composed of various nutrients, including carbohydrates, nitrogen sources, minerals, and vitamins.

Furthermore, the cell preparation processing method provided in this embodiment is realized through an integrated cell preparation processing device, which is arranged in a sterile environment. The cell preparation device includes the cell culture container that is sealed in a sterile environment and an input tube. One end of the input tube is connected to the cell culture container, and the other end of the input tube can be connected to a collection bag (used to store a cell solution such as the cell suspension or other reagents). In this way, the introduction of the to-be-cultured cell suspension into the cell culture container, as well as the subsequent introduction of an activation reagent and the like, can be carried out in a sealed and sterile environment. This prevents the quality of the cell suspension or reagents from being compromised by external environmental factors, thereby improving the quality of cell culture.

The cell culture container can be a rotatable centrifuge container, a bottle, a canister, or a piston-type centrifuge tube. In an embodiment, during the cell expansion process, the cell culture container can be shaken, rotated, or vibrated to improve the culturing yield of the to-be-cultured cell suspension and accelerate the incubation of the to-be-cultured cell suspension. For example, during a culturing cycle, the cell culture container can be intermittently rotated left and right at a preset speed, which can range from 10 to 400 revolutions per minute (rpm). The culturing cycle can last for 24 hours. The intermittent rotation can be set, for example, to occur for a preset duration at hourly intervals throughout the culturing cycle. The preset rotation speed can be adjusted based on a cell density of the to-be-cultured cell suspension.

S20, performing washing and concentration processing on the cultured cell suspension in the cell culture container to obtain a concentrated cell suspension.

It is understood that a washing solution bag 2 is connected to the cell culture container, and the washing solution bag contains a washing solution, which can be a mixture of physiological saline and 0.5% human serum albumin (HSA). The washing solution is poured into the cell culture container with two inlets/outlets. The washing and concentration process is performed to remove impurities from the cultured cell suspension, thereby extracting a cell suspension with higher concentration and higher purity. During the expansion of the T cells or the T cell subsets in the to-be-cultured suspension, some non-cellular impurities may be generated. The subsequent washing and concentration step removes the impurities, thereby obtaining the concentrated cell suspension containing primarily the T cells and/or the T cell subsets. An intermediate product bag 5 can be used as a buffer or temporary storage container during this washing and concentration process.

S30, dispensing the concentrated cell suspension from the cell culture container into a plurality of preparation bags.

After the washing and concentration processing is performed on the cultured cell suspension in the cell culture container, the obtained concentrated cell suspension contains primarily the T cells and/or the T cell subsets; furthermore, the concentrated cell suspension in the cell culture container can be dispensed into multiple preparation bags.

Furthermore, directly dispensing the concentrated cell suspension into the preparation bags may lead to a decrease of a cell viability over time. Therefore, before dispensing the concentrated cell suspension into the preparation bags, a cryopreservation solution is introduced into the cell culture container containing the concentrated cell suspension, thereby freezing the concentrated cell suspension through the cryopreservation solution. In this way, the cryopreservation solution and the concentrated cell suspension can be mixed and then dispensed into the preparation bags, thereby maintaining the cell viability.

Furthermore, on one hand, directly mixing the cryopreservation solution and the concentrated cell solution and then dispensing the mixture into the preparation bags may result in varying dispensing sizes in each preparation bag. On the other hand, there may be specific dispensing requirements (such as the need to dispense the mixture into preparation bags of different sizes). Therefore, a quantitative tube is provided to more accurately dispense the mixture of the cryopreservation solution and the concentrated cell solution into the corresponding preparation bags. That is, a volume of the mixture of the cryopreservation solution and the concentrated cell solution is measured using the quantitative tube, and then the mixture is dispensed into the corresponding preparation bags.

In one embodiment, during the process of dispensing the concentrated cell suspension into each of the preparation bags 4, a replacement solution bag 3 can be connected to the cell culture container to match the cell storage environment. The replacement solution bag contains a replacement solution such as the cryopreservation solution and a resuspension solution, which is used to replace components in the concentrated cell suspension. The replacement solution is delivered into the cell culture container via the delivery device (e.g., a peristaltic pump 10), allowing the replacement solution to suspend the cells in the concentrated cell suspension. The cell culture container can be rotated left and right to mix the replacement solution and the concentrated cell suspension evenly before the mixture of the replacement solution and the concentrated cell suspension is dispensed into the preparation bags. The dispensing process involves distributing the liquid from the cell culture container into each preparation bag according to the preparation requirements, filling each preparation bag with the corresponding volume of liquid.

All tubing paths in the present disclosure can be equipped with sensors for monitoring the tubing paths according to needs, such as a bubble sensor for detecting a flow direction and a flow rate of liquid in the corresponding tubing path, a pressure sensor for detecting a pressure in the corresponding tubing path, a flow sensor for detecting a transmitted flow rate, and a photoelectric sensor for detecting the volume of the liquid in the cell culture container. All the tubing paths in the present disclosure can be respectively equipped with an on-off valve 7 for opening and closing the corresponding tubing path according to needs, such as using a solenoid valve for opening and closing the inlet/outlet of various liquid bags.

In an embodiment, the cryopreservation solution or the resuspension solution and the concentrated cell suspension in the cell culture container 12 are quantitatively formulated through a quantitative tube 11, such that the quantitatively formulated cryopreservation solution and concentrated cell suspension can be dispensed into respective preparation bags. During the quantitative formulation process, a bubble sensor 8/liquid sensor is arranged on an inlet tube and an outlet tube of the quantitative tube. The moments when liquid enters and exits the quantitative tube are detected by the bubble sensor/liquid sensor, defined as an initial moment and an end moment respectively. Based on a fixed capacity of the quantitative tube and a time difference between the initial moment and the end moment, a constant flow rate of the liquid delivered by the peristaltic pump operating at a constant speed during this period can be determined. Since the working time required to fill each preparation bag can be calculated by dividing the capacity of the preparation bag by the constant flow rate, the solution can be accurately dispensed into each preparation bag using the constant flow rate. The moment when the liquid enters each preparation bag is detected through the sensor arranged on each preparation bag, thereby improving the dispensing accuracy for each preparation bag. Before detecting the moments when the liquid enters and exits the quantitative tube through the bubble sensor/liquid sensor, bubble removal treatment can be performed on the liquid entering the quantitative tube. Initially, the inlet of the quantitative tube may be positioned below the outlet thereof, eliminating air bubbles from the quantitative tube using the principle of bubble rising. After the quantitative tube is completely filled, the quantitative tube can be switched to allow the inlet to be positioned above the outlet thereof, thereby maintaining the quantitative tube in a liquid-filled state.

In this embodiment, the cell culture, the cell washing and concentration processing, and the preparation dispensing are all performed within the same cell culture container, which avoids dispersed arrangement of the devices and thus reduces space occupation. By performing these steps sequentially within the same cell culture container, the entire process from the cell culture to the cell preparation can be carried out in a sealed sterile environment without the need for cell suspension transfer, thereby improving the quality of the cell culture. Furthermore, this sequential and integrated process reduces manual involvement and enables intelligent execution from the cell culture to the preparation dispensing, significantly improving the efficiency of the cell culture.

In an embodiment, in step S10, namely the performing cell expansion processing on a to-be-cultured cell suspension in a cell culture container to obtain a cultured cell suspension includes steps as follows.

Introducing an activation reagent into the cell culture container, wherein the activation reagent is one or more selected from the group consisting of: an agonist antibody CD3, an agonist antibody CD28, a cytokine recombinant protein, a costimulatory molecule, a lectin, an ionophore, a synthetic molecule, an antigen-presenting cell, an artificial antigen-presenting cell, and a feeder cell.

It is understood that a portion of the activation reagent can be pre-coated in the cell culture container. A reagent bag containing the activation reagent can be connected to the inlet/outlet of the cell culture container. The activation reagent is then delivered into the cell culture container along the connected tubing path via the delivery device (such as a peristaltic pump). It is noted that the tubing path for the to-be-cultured cell suspension into the cell culture container and the tubing path for the activation reagent into the cell culture container may largely overlap (when using a single peristaltic pump for delivery). A three-way valve is used to switch the flow direction of the to-be-cultured cell suspension and the activation reagent. Alternatively, the tubing path for the to-be-cultured cell suspension into the cell culture container and the tubing path for the activation reagent into the cell culture container may be separated from each other by different peristaltic pumps.

As mentioned above, the cell expansion processing is used to induce the proliferation of the to-be cultured cell suspension, and the reagent used to induce T-cell proliferation is the activation reagent. In this embodiment, the cell expansion can be achieved by performing an activation reaction between a single activation reagent and the to-be-cultured cell suspension, or the activation reaction between multiple activation reagents and the to-be-cultured cell suspension. The total amount of the introduced activation reagent can be determined based on the total volume of the to-be-cultured cell suspension in the cell culture container. For example, when the to-be-cultured cell suspension contains 4 × 10^6 cells/mL, the total amount of the introduced activation reagent may be 8 µg/ml; when the to-be-cultured cell suspension contains 3× 10^6 cells/mL, the total amount of the introduced activation reagent may be 5 µg/mL. It should be noted that although introducing the activation reagent can induce faster cell proliferation, an excessive amount of activation reagent may lead to overcrowding of cells in the cell culture container, resulting in lower cell viability and reduced cell activity. Therefore, in this embodiment, the condition of introducing 5 µg/mL activation reagent for the to-be-cultured cell suspension with 3×10^6 cells/mL is used as the reference standard.

In this way, before the activation reagent is introduced into the cell culture container, the total amount of the required activation reagent can be calculated based on the total volume of the to-be-cultured cell suspension in the cell culture container. For example, a ratio between the total volume of the to-be-cultured cell suspension and the activation reagent in the above reference standard is determined as a set ratio, such that the total amount of the activation reagent can be determined based on the total volume of the to-be-cultured cell suspension in the cell culture container and the set ratio. Then, the type of the activation reagent required for the current round of cell expansion processing is determined, such as one or more of the types mentioned above; if only one type of the activation reagent is included, then the above determined total amount of the activation reagent is directly introduced into the cell culture container; if multiple types of activation reagents are included, then the respective amount of each type of the activation reagent to be introduced can be determined based on the above determined total amount and types.

Furthermore, when the cell expansion processing in performed on the to-be-cultured cell suspension in the cell culture container for the first time, in addition to introducing the activation reagent, glucose is also required. A culture medium 6 containing the activation reagent and the glucose can be introduced into the cell culture container, wherein the glucose provides nutrition for the to-be-cultured cell suspension. In the subsequent cell expansion processing performed on the to-be-cultured cell suspension in the cell culture container, the culture medium containing only the glucose can be introduced without the need to introduce the activation reagent, as the cells are in an activated state after activation and may continue to expand.

Performing a shaking and binding operation on the cell culture container containing the to-be-cultured cell suspension and the activation reagent to obtain the cultured cell suspension.

After the activation reagent is introduced into the cell culture container, the shaking and binding operation is performed on the cell culture container containing the to-be-cultured cell suspension and the activation reagent. For this purpose, a holding device for holding the cell culture container (such as a robotic arm) can be pre-installed on the device. Alternatively, a mechanical shaking apparatus can be arranged on the holding device that secures the cell culture container to perform the shaking operation. This fully mixes the activation reagent and the to-be-cultured cell suspension and induce the proliferation of the to-be-cultured cell suspension, resulting in the cultured cell suspension. It should be noted that the shaking and binding operation can be initiated either immediately upon the starting of the introduction of the activation reagent or after the introduction of the activation reagent is complete, which is not limited herein.

In an embodiment, the shaking and binding operation can be performed in a manner combining swinging with right-left rotations.

In an embodiment, the performing a shaking and combining operation on the cell culture container containing the to-be-cultured cell suspension and the activation reagent to obtain the cultured cell suspension includes steps as follows.

During the process of shaking and binding operation, detecting a cell density of the cells in the cell culture container by a density acquisition device to determine the cell density of the cells in the cell culture container.

It is understood that after the activation reagent is introduced into the cell culture container, the shaking and binding operation is performed to fully mix the activation reagent and the to-be-cultured cell suspension and then induce the proliferation of the to-be-cultured cell suspension. At this point, it is necessary to determine when to stop the shaking and binding operation. Therefore, in this embodiment, the density acquisition device is configured to detect the cell density in the cell culture container, thereby determining whether the cell density in the cell culture container meets the corresponding requirements. It should be noted that cell density detection can be performed either immediately upon the starting of the shaking and binding operation, or after a delay of a specified duration (such as 5 s, 10 s, or 15 s). The cell density reflects a number of the cells in the cell culture container.

In one embodiment, a sampling port is provided adjacent to the inlet/outlet of the cell culture container. A small amount of liquid from the cell culture container is extracted through the sampling port and flows into the density acquisition device, where the cell density of the sampled liquid is detected.

As mentioned above, the cell expansion process aims to significantly increase the number of the T cells and the T cell subsets to meet the cell number required for the cell therapy. Therefore, whether the current expanded number of the cells meets the cell number required for the cell therapy can be determined based on the cell density.

When the detected cell density reaches or exceeds a preset density threshold, stopping the shaking and binding operation to obtain the cultured cell suspension.

It is understood that the preset density threshold can be determined based on factors such as the volume of the cell culture container and the cell number required for the cell therapy. In an embodiment, after the cell density in the cell culture container is detected by the density acquisition device and the cell density is determined, the cell density can be compared with the preset density threshold. If the detected cell density reaches or exceeds the preset density threshold, the shaking and binding operation is stopped, and the solution in the current cell culture container is identified as the cultured cell suspension. If the cell density is less than the preset density threshold, it indicates that the number of the cells in the cell culture container does not meet the cell number required for the cell therapy. The shaking and binding operation continues until the detected cell density reaches or exceeds the preset density threshold. At this point, the shaking and binding operation is stopped, and the solution in the current cell culture container is identified as the cultured cell suspension.

In an embodiment, the density acquisition device includes an image acquisition device and a cell identification device.

The detecting a cell density of the cells in the cell culture container by a density acquisition device to determine the cell density of the cells in the cell culture container includes steps as follows.

Acquiring an image of the cells in the cell culture container by the image acquisition device to obtain a cell image.

It is understood that, the image acquisition device can be a camera, video camera, or miniature monitor installed on the equipment, and the image acquisition device can be positioned above the cell culture container. The cell image refers to the image containing the cells captured by the image acquisition device from the cell culture container.

Performing cell counting and identification on the cell image by the cell identification device to determine a number of the cells contained in the cell image.

It is understood that the cell counting and identification involves identifying the cells in the cell image and then calculating the number of the cells contained in the cell image. In an embodiment, after the cell image of the cells in the cell culture container is obtained using the image acquisition device, the cell image is then identified by the cell identification device to determine the cells contained in the image. During the process, the cells contained in the cell image can be marked, and the number of marks can be determined as the number of the cells in the cell image.

In an embodiment, the performing cell counting and identification on the cell image through the cell identification device to determine the number of the cells in the cell image includes steps as follows.

Inputting the cell image into the cell identification model and extracting cell shape features from the cell image using the cell identification model. The cell identification model is a trained neural network model used to identify cell regions in the input cell image. The cell identification model is obtained through continuous convergence by deep learning of the cell shape features. The cell shape features are presented by edges of the cell and an overall shape enclosed by the edges. During the process of extracting the cell shape features, image preprocessing such as grayscale processing and edge enhancement can be performed on the cell image.

Identifying the cells in the cell image using the cell identification model based on the extracted cell shape features to obtain the cell region having the cell shape features. The cell region can correspond to a single cell, or correspond to overlapping or adjacent cells.

Counting a number of the identified cell regions to obtain the number of the cells in the cell image. During the counting process, the number of the cells in overlapping or adjacent regions is correspondingly calculated to determine the actual number of the cells. For example, if an overlapping region falls within the size range of one to two cells, it may be counted as containing two cells, which enables more accurate identification of the number of the cells, thereby improving the identification reliability of the number of the cells.

The network structure of the neural network model can be the You Only Look Once (YOLO), the Visual Geometry Group (VGG), or the Residual Networks (ResNets). The cell shape features includes the size, shape, clustering, and stacking characteristics of the cells.

The YOLO network structure is an algorithmic network structure that provides real-time object detection. The YOLO algorithm utilizes a convolutional neural network for a single forward propagation to detect objects, enabling real-time detection and identification of various objects within images.

The VGG network structure employs three 3x3 convolutional kernels to replace a 7x7 convolutional kernel and two 3x3 convolutional kernels to replace a 5x5 convolutional kernel. The main purpose of this configuration is to increase a depth of the network while maintaining the same receptive field, thereby improving the performance of the neural network to a certain extent.

The ResNets structure allows certain layers of the neural network to skip connections to the next layer of neurons, establishing connections across layers. This configuration weakens the strong interdependence between consecutive layers, helps mitigate the vanishing gradient and exploding gradient problems, and ensures good network performance even when training deeper networks.

Obtaining image parameters corresponding to the cell image, and determining the cell density based on the number of the cells and the image parameters.

It is understood that, the image parameters refer to the parameters required for density conversion of the number of the cells, such as a resolution of the cell image. In an embodiment, after the cell counting and identification are performed on the cell image through the cell identification device to determine the number of the cells in the cell image, the image parameters corresponding to the cell image are obtained. Based on the cell number and the image parameters, the cell density is determined. The image parameters include an image area and an image depth of field. By obtaining the set image area and the image depth of field, an image volume of the obtained cell image is determined. The cell density is obtained by dividing the number of the cells by the image volume.

In an embodiment, step S20, that is, the performing washing and concentration processing on the cultured cell suspension in the cell culture container to obtain a concentrated cell suspension includes steps as follows.

Washing the cultured cell suspension in the cell culture container to obtain a mixed cell suspension.

It is understood that in this embodiment, the cultured cell suspension is at first washed to preliminarily remove non-cellular impurities contained in the cultured cell suspension, thereby obtaining the mixed cell suspension. In this embodiment, the mixed cell suspension can be obtained by introducing a buffer solution into the cell culture container containing the cultured cell suspension and performing a centrifugal washing operation on the cell culture container containing the buffer solution and the cultured cell suspension.

The buffer solution is used for washing the cultured cell suspension of non-cellular impurities. A volume of the introduced buffer solution is determined based on the volume of the cultured cell suspension. For example, the cultured cell suspension of up to 250 mL can be washed with 800 mL of the buffer solution, while 250 mL to 500 mL of the cultured cell suspension can be washed with 1200 mL of the buffer solution. In an embodiment, when the cell culture container contains 250 mL of the cultured cell suspension, two washes are used, each using 400 mL of the buffer solution. Introducing the buffer solution in batches can fully utilize the washing effect of each introduction of the buffer solution on the cultured cell suspension, improving the efficiency of impurity removal, and thus improving the purity of the mixed cell suspension.

Concentrating the mixed cell suspension in the cell culture container to obtain the concentrated cell suspension.

It is understood that, the cell concentration in the mixed cell suspension obtained after the centrifugal washing operation is performed on the cultured cell suspension with the buffer solution is not high enough, and the mixed cell suspension may still contain a small amount of the non-cellular impurities. Therefore, after the mixed cell suspension is obtained, a centrifugal extraction operation is performed on the cell culture container containing the mixed cell suspension to separate the non-cellular impurities from the mixed cell suspension, thereby obtaining the concentrated cell suspension containing only target cells. During the centrifugal extraction operation, the cell suspension with a higher concentration can be extracted to an upper layer, while the cell suspension with a lower concentration or impurities can be extracted to a lower layer, which improves the cell concentration while further removing the non-cellular impurities. At this point, the concentrated cell suspension in the cell culture container ultimately contains only T cells and/or T cell subsets.

In an embodiment, after step S20, that is, after the performing washing and concentration processing on the cultured cell suspension in the cell culture container to obtain a concentrated cell suspension, the method further includes steps as follows.

Performing sampling test on the concentrated cell suspension to determine whether the concentrated cell suspension satisfies preset preparation conditions.

It is understood that, after the washing and concentration processing is performed to obtain the concentrated cell suspension, and before dispensing the concentrated cell suspension into multiple preparation bags, the sampling test is performed on the concentrated cell suspension, that is, a cell density test and a cell viability test are performed on the concentrated cell suspension. This ensures a survival rate and a cell activity of the cells in the concentrated cell suspension dispensed into the preparation bags, thereby guaranteeing the quality of the cells in the concentrated cell suspension. The preset preparation conditions include whether the cell density in the concentrated cell suspension is greater than or equal to the preset density threshold, and whether the cell viability is greater than or equal to a preset viability threshold. The preset density threshold and the preset viability threshold can be set according to the cell density, the number of the cells, and the cell viability required for the cell therapy.

When the concentrated cell suspension satisfies the preset preparation conditions, the concentrated cell suspension is dispensed into the multiple preparation bags.

In an embodiment, after the sampling test is performed on the concentrated cell suspension, if the concentrated cell suspension satisfies the preset preparation conditions, the concentrated cell suspension is dispensed into the multiple preparation bags. If the concentrated cell suspension does not satisfy the preset preparation conditions, the washing and concentration processing is repeated. The re-processed concentrated cell suspension is then retested against the preset preparation conditions. When it is determined that the re-processed concentrated cell suspension satisfies the preset preparation conditions, the re-processed concentrated cell suspension is dispensed into the multiple preparation bags.

It should be understood that the sequence numbers of the steps in the above embodiments do not indicate the order of execution. The execution order of each process should be determined by its function and inherent logic, and should not constitute any limitation on the implementation process of the embodiments of the present disclosure.

In an embodiment, a cell preparation processing device is provided, including:
a cell expansion module, configured to perform cell expansion processing on a to-be-cultured cell suspension in a cell culture container to obtain a cultured cell suspension;
a washing and concentration module, configured to perform washing and concentration processing on the cultured cell suspension in the cell culture container to obtain a concentrated cell suspension; and
a first preparation dispensing module, configured to dispense the concentrated cell suspension from the cell culture container into a plurality of preparation bags.

Furthermore, the cell expansion module includes:
an activation reagent introduction sub-module, configured to introduce an activation reagent into the cell culture container; wherein the activation reagents are one or more of selected from the group consisting of: an agonist antibody CD3, an agonist antibody CD28, a cytokine recombinant protein, a costimulatory molecules, a lectin, an ionophore, a synthetic molecule, an antigen-presenting cell, an artificial antigen-presenting cell, and a feeder cell; and
a solution shaking and binding sub-module, configured to perform a shaking and binding operation on the cell culture container containing the to-be-cultured cell suspension and the activation reagent, thereby obtaining the cultured cell suspension.

Furthermore, the solution shaking and binding sub-module includes:
a density detection unit, configured to detect a cell density of cells in the cell culture container through a density acquisition device to determine the cell density of the cells in the cell culture container during the shaking and binding operation; and
a density comparison unit, configured to stop the shaking and binding operation to obtain the cultured cell suspension when the detected cell density reaches or exceeds a preset density threshold.

Furthermore, the density detection unit includes:
an image acquisition sub-unit, configured to acquire an image of cells in the cell culture container by the image acquisition device to obtain a cell image;
a cell identification sub-unit, configured to perform cell counting and identification on the cell image by the cell identification device to determine the number of cells in the cell image; and
a density conversion sub-unit, configured to obtain image parameters corresponding to the cell image and determine the cell density based on the number of the cells and the image parameters.

Furthermore, the washing and concentration module includes:
a washing processing unit, configured to wash the cultured cell suspension in the cell culture container to obtain a mixed cell suspension; and
a concentration processing unit, configured to concentrate the mixed cell suspension in the cell culture container to obtain the concentrated cell suspension.

Furthermore, the washing processing unit includes:
a buffer solution introduction sub-unit, configured to introduce a buffer solution into the cell culture container containing the cultured cell suspension; and
a centrifugal washing sub-unit, configured to perform a centrifugal washing operation on the cell culture container containing the buffer solution and the cultured cell suspension to obtain the mixed cell suspension.

Furthermore, the concentration processing unit includes:
a centrifugal extraction sub-unit, configured to perform a centrifugal extraction operation on the cell culture container containing the mixed cell suspension to remove non-cellular impurities from the mixed cell suspension, thereby obtaining the concentrated cell suspension containing only target cells; wherein the target cells include T cells and/or T cell subsets.

Furthermore, the first preparation dispensing module includes:
a cryopreservation solution introduction unit, configured to introduce a cryopreservation solution into the cell culture container containing the concentrated cell suspension; and
a preparation dispensing unit, configured to mix the cryopreservation solution and the concentrated cell suspension, and dispense the mixture from the cell culture container into the preparation bags.

Furthermore, the preparation dispensing unit includes:
a preparation dispensing sub-unit, configured to perform quantitative formulation on the cryopreservation solution and the concentrated cell suspension in the cell culture container through a quantitative tube, thereby dispensing the quantitatively formulated cryopreservation solution and concentrated cell suspension into respective preparation bags.

Furthermore, the cell preparation processing device further includes:
a sampling testing module, configured to perform sampling test on the concentrated cell suspension to determine whether the concentrated cell suspension satisfies preset preparation conditions; and
a second preparation dispensing module, configured to dispense the concentrated cell suspension into the multiple preparation bags when the concentrated cell suspension satisfies the preset preparation conditions.

The present disclosure further provides a cell preparation processing method system, including a cell culture container and a controller connected to the cell culture container, wherein the controller is configured to execute the cell preparation processing method.

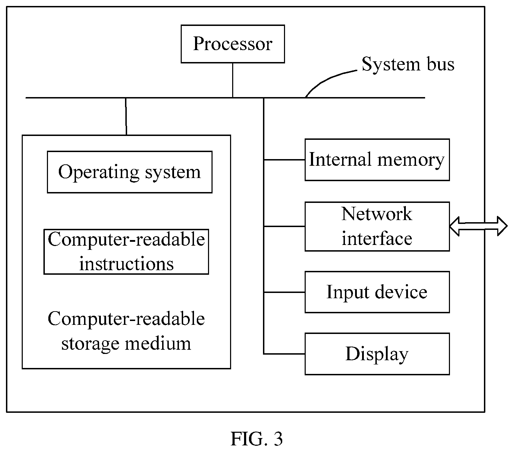

Regarding the specific definitions of the cell preparation processing system, the controller, and various units and modules of the ell preparation processing system, reference may be made to the definitions provided above for the cell preparation processing method, which will not be repeated herein. Each module in the controller can be implemented in whole or in part through software, hardware, or a combination thereof. It is understood that, as shown in FIG. 3, the controller includes a processor, a memory, network interface, and database connected via a system bus. Each module of the controller can be embedded in or independent of the processor in the form of hardware, or stored in the memory in the form of software, such that the processor can call and execute the operations corresponding to each module. The processor provides computational and control capabilities. The memory includes a computer-readable storage medium and an internal memory. The computer-readable storage medium stores an operating system, computer-readable instructions, and a database. The internal memory provides an environment for the operation of the operating system and computer-readable instructions in the computer-readable storage medium. The database is used to store the data used in the cell preparation processing method described in the above embodiments. The network interface is used for network communication with external terminals. The computer-readable instructions, when executed by the processor, implement the cell preparation processing method. The computer-readable storage medium provided in this embodiment includes a non-volatile computer-readable storage medium and a volatile computer-readable storage medium.

In one embodiment, one or more computer-readable storage media storing computer-readable instructions are provided. The computer-readable storage medium provided in this embodiment includes both a non-volatile computer-readable storage medium and a volatile computer-readable storage medium. When being executed by one or more processors, the computer-readable instructions stored on the computer-readable storage medium enable the one or more processors to implement the above cell preparation processing method.

Those skilled in the art may understand that all or some of the processes of the method in the above embodiments may be implemented by instructing related hardware by using computer-readable instructions. The computer-readable instructions may be stored in a non-volatile computer-readable storage medium or a volatile computer-readable storage medium. When the computer-readable instructions are executed, the processes in the above method embodiments may be implemented. Any reference to the memory, storage, database, or other media used in the embodiments provided in the present disclosure may include a non-volatile and/or volatile memory. The non-volatile memory may include a read-only memory (ROM), a programmable ROM (PROM), an electrically programmable ROM (EPROM), an electrically erasable programmable ROM (EEPROM), or a flash memory. The volatile memory may include a random access memory (RAM) or an external cache memory. By way of illustration instead of limitation, the RAM is available in a plurality of forms, such as a static RAM (SRAM), a dynamic RAM (DRAM), a synchronous DRAM (SDRAM), a double data rate SDRAM (DDRSDRAM), an enhanced SDRAM (ESDRAM), a synchlink (Synchlink) DRAM (SLDRAM), a Rambus (Rambus) direct RAM (RDRAM), a direct Rambus dynamic RAM (DRDRAM), and a Rambus dynamic RAM (RDRAM).

Those skilled in the art may clearly understand that, for convenience and brevity of description, only the division of the above functional units or modules is used as an example for description. In actual applications, the above functions may be allocated to different functional units or modules for implementation as required, that is, an internal structure of the device is divided into different functional units or modules to implement all or some of the functions described above.

The above descriptions are only optional embodiments of the application, and do not limit the scope of the patents of the present disclosure. All the equivalent structural transformations made by the content of the specification and drawings of the present disclosure under the creative concept of the present disclosure, or directly/indirectly used in other related technical fields are all comprised in the protection scope of the patents of the present disclosure.

## Claims

1. A cell preparation processing method, comprising:
performing cell expansion processing on a to-be-cultured cell suspension in a cell culture container to obtain a cultured cell suspension;
performing washing and concentration processing on the cultured cell suspension in the cell culture container to obtain a concentrated cell suspension; and
dispensing the concentrated cell suspension from the cell culture container into a plurality of preparation bags.

2. The cell preparation processing method according to claim 1, wherein the performing cell expansion processing on a to-be-cultured cell suspension in a cell culture container to obtain a cultured cell suspension comprises:
introducing an activation reagent into the cell culture container, wherein the activation reagent is one or more selected from the group consisting of: an agonist antibody CD3, an agonist antibody CD28, a cytokine recombinant protein, a costimulatory molecule, a lectin, an ionophore, a synthetic molecule, an antigen-presenting cell, an artificial antigen-presenting cell, and a feeder cell; and
performing a shaking and binding operation on the cell culture container containing the to-be-cultured cell suspension and the activation reagent to obtain the cultured cell suspension.

3. The cell preparation processing method according to claim 2, wherein the performing a shaking and binding operation on the cell culture container containing the to-be-cultured cell suspension and the activation reagent to obtain the cultured cell suspension comprises:
during the shaking and binding operation, detecting a cell density of cells in the cell culture container by a density acquisition device to determine the cell density of the cells in the cell culture container; and
when the detected cell density reaches or exceeds a preset density threshold, stopping the shaking and binding operation to obtain the cultured cell suspension.

4. The cell preparation processing method according to claim 3, wherein the density acquisition device comprises an image acquisition device and a cell identification device;
and wherein the detecting a cell density of cells in the cell culture container by a density acquisition device to determine the cell density of the cells in the cell culture container comprises:
acquiring an image of the cells in the cell culture container by the image acquisition device to obtain a cell image;
performing cell counting and identification on the cell image by the cell identification device to determine a number of cells in the cell image; and
obtaining image parameters corresponding to the cell image, and determining the cell density based on the number of the cells and the image parameters.

5. The cell preparation processing method according to claim 1, wherein the performing washing and concentration processing on the cultured cell suspension in the cell culture container to obtain a concentrated cell suspension comprises:
washing the cultured cell suspension in the cell culture container to obtain a mixed cell suspension; and
concentrating the mixed cell suspension in the cell culture container to obtain the concentrated cell suspension.

6. The cell preparation processing method according to claim 5, wherein the washing the cultured cell suspension in the cell culture container to obtain a mixed cell suspension comprises:
introducing a buffer solution into the cell culture container containing the cultured cell suspension; and
performing a centrifugal washing operation on the cell culture container containing the buffer solution and the cultured cell suspension to obtain the mixed cell suspension.

7. The cell preparation processing method according to claim 5, wherein the concentrating the mixed cell suspension in the cell culture container to obtain the concentrated cell suspension, comprises:
performing a centrifugal extraction operation on the cell culture container containing the mixed cell suspension to remove non-cellular impurities therefrom, thereby obtaining the concentrated cell suspension containing only target cells, wherein the target cells comprise T cells and/or T cell subsets.

8. The cell preparation processing method according to claim 1, wherein the dispensing the concentrated cell suspension from the cell culture container into a plurality of preparation bags comprises:
introducing a cryopreservation solution into the cell culture container containing the concentrated cell suspension; and
mixing the cryopreservation solution and the concentrated cell suspension and dispensing the mixture from the cell culture container into the plurality of preparation bags.

9. The cell preparation processing method according to claim 8, wherein the dispensing the mixture from the cell culture container into the plurality of preparation bags comprises:
performing quantitative formulation on the cryopreservation solution and
the concentrated cell suspension in the cell culture container through a quantitative tube, thereby dispensing the quantitatively formulated cryopreservation solution and concentrated cell suspension into respective preparation bags.

10. The cell preparation processing method according to claim 1, wherein the performing washing and concentration processing on the cultured cell suspension in the cell culture container to obtain a concentrated cell suspension comprises:
performing sampling test on the concentrated cell suspension to determine whether the concentrated cell suspension satisfies preset preparation conditions; and
dispensing the concentrated cell suspension into the plurality of preparation bags when the concentrated cell suspension satisfies the preset preparation conditions.

11. A cell preparation processing device, comprising:
a cell expansion module, configured to perform cell expansion processing on a to-be-cultured cell suspension in a cell culture container to obtain a cultured cell suspension;
a washing and concentration module, configured to perform washing and concentration processing on the cultured cell suspension in the cell culture container to obtain a concentrated cell suspension; and
a first preparation dispensing module, configured to dispense the concentrated cell suspension from the cell culture container into a plurality of preparation bags.

12. The cell preparation processing device according to claim 11, wherein the cell expansion module comprises:
an activation reagent introduction sub-module, configured to introduce an activation reagent into the cell culture container, wherein the activation reagent is one or more selected from the group consisting of: an agonist antibody CD3, an agonist antibody CD28, a cytokine recombinant protein, a costimulatory molecule, a lectin, an ionophore, a synthetic molecule, an antigen-presenting cell, an artificial antigen-presenting cell, and a feeder cell; and
a solution shaking and binding sub-module, configured to perform a shaking and binding operation on the cell culture container containing the to-be-cultured cell suspension and the activation reagent to obtain the cultured cell suspension.

13. The cell preparation processing device according to claim 12, wherein the solution shaking and binding sub-module comprises:
a density detection unit, configured to, during the shaking and binding operation, detect a cell density of cells in the cell culture container by a density acquisition device to determine the cell density of the cells in the cell culture container; and
a density comparison unit, configured to stop the shaking and binding operation to obtain the cultured cell suspension when the detected cell density reaches or exceeds a preset density threshold.

14. The cell preparation processing device according to claim 13, wherein the density detection unit comprises:
an image acquisition sub-unit, configured to acquire an image of the cells in the cell culture container by the image acquisition device to obtain a cell image; and
a cell identification sub-unit, configured to perform cell counting and identification on the cell image by the cell identification device to determine the number of cells in the cell image; and
a density conversion sub-unit, configured to obtain image parameters corresponding to the cell image and determine the cell density based on the number of the cells and the image parameters.

15. The cell preparation processing device according to claim 11, wherein the washing and concentration module comprises:
a washing processing unit, configured to wash the cultured cell suspension in the cell culture container to obtain a mixed cell suspension; and
a concentration processing unit, configured to concentrate the mixed cell suspension in the cell culture container to obtain the concentrated cell suspension.

16. The cell preparation processing device according to claim 15, wherein the washing processing unit comprises:
a buffer solution introduction sub-unit, configured to introduce a buffer solution into the cell culture container containing the cultured cell suspension; and
a centrifugal washing sub-unit, configured to perform a centrifugal washing operation on the cell culture container containing the buffer solution and the cultured cell suspension to obtain the mixed cell suspension.

17. The cell preparation processing device according to claim 15, wherein the concentration processing unit comprises:
a centrifugal extraction sub-unit, configured to perform a centrifugal extraction operation on the cell culture container containing the mixed cell suspension to remove non-cellular impurities from the mixed cell suspension, thereby obtaining the concentrated cell suspension containing only target cells, wherein the target cells comprise T cells and/or T cell subsets.

18. The cell preparation processing device according to claim 11, wherein the cell preparation processing device further comprises:
a sampling test module, configured to perform sampling test on the concentrated cell suspension to determine whether the concentrated cell suspension satisfies preset preparation conditions; and
a second preparation dispensing module, configured to dispense the concentrated cell suspension into the plurality of preparation bags when the concentrated cell suspension satisfies the preset preparation conditions.

19. A cell preparation processing method system, comprising a cell culture container and a controller connected to the cell culture container, wherein the controller is configured to execute the following cell preparation processing method:
performing cell expansion processing on a to-be-cultured cell suspension in a cell culture container to obtain a cultured cell suspension;
performing washing and concentration processing on the cultured cell suspension in the cell culture container to obtain a concentrated cell suspension; and
dispensing the concentrated cell suspension from the cell culture container into a plurality of preparation bags.

20. One or more computer-readable storage media storing computer-readable instructions, wherein when the computer-readable instructions are executed by one or more processors, the one or more processors are caused to perform the following steps:
performing cell expansion processing on a to-be-cultured cell suspension in a cell culture container to obtain a cultured cell suspension;
performing washing and concentration processing on the cultured cell suspension in the cell culture container to obtain a concentrated cell suspension; and
dispensing the concentrated cell suspension from the cell culture container into a plurality of preparation bags.
